# EUROPEAN PATENT SPECIFICATION

(11) **EP 3 645 749 B1**
(45) Date of publication and mention of the grant of the patent: **02.03.2022**
(21) Application number: 18824489.1
(22) Date of filing: 27.06.2018
(51) Int. Cl.: C12Q 1/70

(54) **MULTIPLEXED FLUOROMETRIC MEASUREMENTS WITH DROPLET PCR SYSTEMS**
MULTIPLEX-FLUOROMETRISCHE MESSUNGEN MIT TRÖPFCHEN-PCR-SYSTEMEN
MESURES FLUORIMÉTRIQUES MULTIPLEXÉES AVEC SYSTÈMES DE PCR EN GOUTTELETTES

(30) Priority: 28.06.2017 US 201762526272 P; 10.01.2018 US 201862615560 P
(43) Date of publication of application: 06.05.2020
(60) Divisional application: 22152874.8
(73) Proprietor: Chromacode, Inc., Carlsbad, California 92008 (US)
(72) Inventor: RAJAGOPAL, Aditya, Carlsbad California 92008 (US)
(74) Representative: HGF
(86) International application number: PCT/US2018/039846
(87) International publication number: WO 2019/006023

(56) References cited:
- WO-A1-2014/149480
- US-A1- 2014 213 471
- US-A1- 2016 108 464
- QUN ZHONG ET AL: "Multiplex digital PCR: breaking the one target per color barrier of quantitative PCR", LAB ON A CHIP, vol. 11, no. 13, 1 January 2011 (2011-01-01), page 2167, XP055109828, ISSN: 1473-0197, DOI: 10.1039/c1lc20126c
- ALEXANDRA S. WHALE ET AL: "Fundamentals of multiplexing with digital PCR", BIOMOLECULAR DETECTION AND QUANTIFICATION, vol. 10, 1 December 2016 (2016-12-01), pages 15-23, XP055504396, ISSN: 2214-7535, DOI: 10.1016/j.bdq.2016.05.002

## Description

### BACKGROUND OF THE INVENTION

Droplet Digital PCR (ddPCR) is a useful method for detection and quantification of nucleic acid targets. The use of labeled oligonucleotide probes enables specific detection of a target present in an emulsion (i.e. droplet). However, ddPCR is limited in the number of targets that can be measured in a single reaction. Current multiplex ddPCR technologies are limited to the measurement of two targets in a single reaction using two distinct color channels.

### SUMMARY OF THE INVENTION

Disclosed herein are methods and compositions for increasing the number of targets that can be measured in a single digital assay (e.g., digital PCR, ddPCR, etc.) by enabling the detection of multiple targets using a single optical channel. In some cases, three, four, or more targets are detected using a single optical channel. This is a key improvement over existing digital multiplexing technologies.

The present invention provides a method for performing a digital assay, comprising: (a) partitioning into a plurality of partitions (i) three or more nucleic acid targets and (ii) a set of nucleic acid probes comprising a first nucleic acid probe, a second nucleic acid probe, and a third nucleic acid probe; (b) amplifying the three or more nucleic acid targets in the partitions; (c) generating N signals from the set of nucleic acid probes, each of which N signals corresponds to the presence of a unique combination of nucleic acid targets of the three or more nucleic acid targets in a partition of the plurality of partitions; and (d) detecting the N signals in a single optical channel; wherein, in (a) the first nucleic acid probe, second nucleic acid probe and third nucleic acid probe are configured such that (i) the concentration of the first nucleic acid probe is about X, the concentration of the second nucleic acid probe is at least about 2X, and the concentration of the third nucleic acid probe is at least about 4X, or (ii) the first nucleic acid probe has an intensity level of about Y, the second nucleic acid probe has an intensity level of at least about 2Y and the third nucleic acid probe has an intensity level of at least about 4Y. In some embodiments, N is 3 or more. In some embodiments, N is 4 or more. In some embodiments, the three or more nucleic acid targets are four or more nucleic acid targets. In some embodiments, the set of nucleic acid probes further comprises a fourth nucleic acid probe. In some embodiments, in (a), the concentration of the fourth nucleic acid probe is at least about 8X. In some embodiments, each of the N signals corresponds to a fluorescence intensity level. In some embodiments, the N signals are generated from the set of nucleic acid probes. In some embodiments, each of the set of nucleic acid probes comprises an identical fluorophore. In some embodiments, each of the set of nucleic acid probes comprises a different fluorophore. In some embodiments, the amplifying comprises polymerase chain reaction. In some embodiments, the amplifying comprises linear amplification. In some embodiments, the amplifying comprises a nucleic acid extension reaction.

Thus in one aspect, provided herein is method for performing a digital assay, comprising: (a) partitioning into a plurality of partitions (i) three or more nucleic acid targets and (ii) a set of nucleic acid probes comprising a first nucleic acid probe, a second nucleic acid probe, and a third nucleic acid probe; (b) amplifying the three or more nucleic acid targets in the partitions; (c) generating N signals from the set of nucleic acid probes, each of which N signals corresponds to the presence of a unique combination of nucleic acid targets of the three or more nucleic acid targets in a partition of the plurality of partitions; and (d) detecting the N signals in a single optical channel; wherein, in (a), the first nucleic acid probe has an intensity level of about Y, the second nucleic acid probe has an intensity level of at least about 2Y, and the third nucleic acid probe has an intensity level of at least about 4Y. In some embodiments, the first nucleic acid probe comprises one fluorophore with an intensity level of about Y, the second nucleic acid probe comprises at least two fluorophores each with an intensity level of about Y, and the third nucleic acid probe comprises at least four fluorophores each with an intensity level of about Y. In some embodiments, the first nucleic acid probe comprises a fluorophore with an intensity level of about Y, the second nucleic acid probe comprises a fluorophore with an intensity level of at least about 2Y, and the third nucleic acid probe comprises a fluorophore with an intensity level of at least about 4Y. In some embodiments, N is 3 or more. In some embodiments, N is 4 or more. In some embodiments, the three or more nucleic acid targets are four or more nucleic acid targets. In some embodiments, the set of nucleic acid probes further comprises a fourth nucleic acid probe. In some embodiments, the fourth nucleic acid probe has an intensity level of 8Y. In some embodiments, the fourth nucleic acid probe comprises a fluorophore with an intensity level of at least about 8 Y. In some embodiments, each of the N signals corresponds to a fluorescence intensity level. In some embodiments, the N signals are generated from the set of nucleic acid probes. In some embodiments, each of the set of nucleic acid probes comprises an identical fluorophore. In some embodiments, each of the set of nucleic acid probes comprises a different fluorophore. In some embodiments, the amplifying comprises polymerase chain reaction. In some embodiments, the amplifying comprises linear amplification. In some embodiments, the amplifying comprises a nucleic acid extension reaction.

In some aspects, provided herein is a method for performing a digital assay, comprising: (a) partitioning three or more nucleic acid targets into a plurality of partitions; (b) amplifying the three or more nucleic acid targets in the partitions; (c) generating N signals each of which corresponds to the presence of a unique combination of nucleic acid targets of the three or more nucleic acid targets in a partition of the plurality of partitions; and (d) detecting the signals in a single optical channel. In some embodiments, N is 3 or more. In some embodiments, N is 4 or more. In some embodiments, the three or more nucleic acid targets are four or more nucleic acid targets. In some embodiments, each signal of the N signals corresponds to a fluorescence intensity level. In some embodiments, (a) further comprises partitioning a set of nucleic acid probes comprising three or more nucleic acid probes into the plurality of partitions. In some embodiments, the N signals are generated from the set of nucleic acid probes. In some embodiments, the set of nucleic acid probes each comprise one or more fluorophores. In some embodiments, the set of nucleic acid probes are provided in (a) at concentrations such that each of the N signals corresponds to the presence of a unique combination of nucleic acid targets of the three or more nucleic acid targets in the partition. In some embodiments, each nucleic acid probe of the set of nucleic acid probes comprises a different number of fluorophores, such that the signal corresponds to the presence of a unique combination of nucleic acid targets of the three or more nucleic acid targets in the partition. In some embodiments, each nucleic acid probe of the set of nucleic acid probes in (a) is provided at a different concentration. In some embodiments, the set of nucleic acid probes comprises a first nucleic acid probe, a second nucleic acid probe, and a third nucleic acid probe. In some embodiments, in (a), the concentration of the first nucleic acid probe is about X, the concentration of the second nucleic acid probe is at least about 2X, and the concentration of the third nucleic acid probe is at least about 4X. In some embodiments, the set of nucleic acid probes further comprises a fourth nucleic acid probe and wherein in (a), the concentration of the fourth nucleic acid probe is at least about 8X. In some embodiments, in (a), the first nucleic acid probe comprises one fluorophore, the second nucleic acid probe comprises at least two fluorophores, and the third nucleic acid probe comprises at least four fluorophores. In some embodiments, the set of nucleic acid probes further comprises a fourth nucleic acid probe and wherein in (a), the fourth nucleic acid probe comprises at least six fluorophores. In some embodiments, each of the set of nucleic acid probes comprises an identical fluorophore. In some embodiments, each of the set of nucleic acid probes comprises a different fluorophore. In some embodiments, the amplifying comprises polymerase chain reaction. In some embodiments, the amplifying comprises linear amplification. In some embodiments, the amplifying comprises a nucleic acid extension reaction.

In some aspects, provided herein is a method of performing a digital assay, comprising: (a) amplifying at least three nucleic acid targets from a sample in a plurality of droplets comprising a set of nucleic acid probes comprising three or more nucleic acid probes labeled with fluorophores capable of being detected in a single optical channel, wherein each nucleic acid probe is complementary to a target of the at least three nucleic acid targets; (b) detecting N signals from the plurality of droplets if one or more of the at least three nucleic acid targets is present, wherein each of the N signals corresponds to a unique combination of one or more of the at least three nucleic acid targets present in the droplet; and (c) determining, from the N signals, the presence or absence of each of the at least three nucleic acid targets in the sample. In some embodiments, N is 3 or more. In some embodiments, N is 4 or more. In some embodiments, the three or more nucleic acid targets are four or more nucleic acid targets. In some embodiments, each of the N signals corresponds to a fluorescence intensity level. In some embodiments, the N signals are generated from the set of nucleic acid probes. In some embodiments, the set of nucleic acid probes are provided in (a) at concentrations such that the signal corresponds to the presence of a unique combination of nucleic acid targets of the three or more nucleic acid targets in the partition. In some embodiments, each nucleic acid probe of the set of nucleic acid probes comprises a different number of fluorophores, such that the signal corresponds to the presence of a unique combination of nucleic acid targets of the three or more nucleic acid targets in the partition. In some embodiments, the set of nucleic acid probes comprise a first nucleic acid probe, a second nucleic acid probe, and a third nucleic acid probe. In some embodiments, in (a), the concentration of the first nucleic acid probe is about X, the concentration of the second nucleic acid probe is at least about 2X, and the concentration of the third nucleic acid probe is at least about 4X. In some embodiments, the set of nucleic acid probes further comprise a fourth nucleic acid probe and wherein in (a), the concentration of the fourth nucleic acid probe is at least about 8X. In some embodiments, the first nucleic acid probe comprises a fluorophore with an intensity level of about X, the second nucleic acid probe comprises a fluorophore with an intensity level of at least about 2X, and the third nucleic acid probe comprises a fluorophore with an intensity level of at least about 4X. In some embodiments, the set of nucleic acid probes further comprises a fourth nucleic acid probe and wherein in (a), the fourth nucleic acid probe comprises a fluorophore with an intensity level of at least about 8X. In some embodiments, the set of nucleic acid probes each comprise one or more fluorophores. In some embodiments, the fluorophores are identical fluorophores. In some embodiments, the fluorophores are different fluorophores. In some embodiments, the amplifying comprises polymerase chain reaction. In some embodiments, the amplifying comprises linear amplification. In some embodiments, the amplifying comprises a nucleic acid extension reaction.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG.** 1 shows a schematic representation of example signal readouts from a multiplex digital assay of the present disclosure where each signal corresponds to a target or combination thereof.
**FIG.** 2 shows a schematic representation of example signal readouts from a multiplex digital assay of the present disclosure where each signal corresponds to a single target.
FIG. 3 shows the results of the experiment described in Example 1, demonstrating the generation of signals which uniquely correspond to each combination of nucleic acid targets which signals are detected in a single optical channel.
**FIG.** 4 shows the results of the experiment described in Example 2, illustrating the detection of signals in a single optical channel generated from a nucleic acid target in a droplet digital PCR reaction at various nucleic acid probe concentrations.
**FIG. 5** shows the results of the experiment described in Example 4, illustrating the detection of six targets across two color channels in a single digital PCR reaction.

### DETAILED DESCRIPTION

Polymerase Chain Reaction (PCR) is a method of exponential amplification of specific target nucleic acid in a reaction mix with a nucleic acid polymerase and primers. Primers are short single stranded oligonucleotides which are complementary to the 3' sequences of the positive and negative strand of the target sequence. The reaction mix is cycled in repeated heating and cooling steps. The heating cycle denatures or splits a double stranded nucleic acid target into single stranded templates. In the cooling cycle, the primers bind to complementary sequence on the template. After the template is primed the nucleic acid polymerase creates a copy of the original template. Repeated cycling exponentially amplifies the target 2 fold with each cycle leading to approximately a billion-fold increase of the target sequence in 30 cycles (Saiki et al 1988).

Digital PCR is a process of partitioning a sample containing one or more targets into a plurality of partitions (e.g., wells, droplets, etc.), performing a PCR reaction in each partition, and recording the fluorescence generated by, for example, a target-specific reporter probe. This is generally performed on a digital PCR instrument that measures the fluorescence from each partition in an optical channel through one or more excitation/emission filter sets.

Frequently, the target-specific nucleic acid probe is a short oligonucleotide complementary to one strand of the amplified target. The probe lacks a 3' hydroxyl and therefore is not extendable by the DNA polymerase. TaqMan (ThermoFisher Scientific) chemistry is a common reporter probe method used for multiplex Real-Time PCR (Holland et al. 1991). The TaqMan oligonucleotide probe is covalently modified with a fluorophore and a quenching tag (i.e., quencher). In this configuration the fluorescence generated by the fluorophore is quenched and is not detected by the real time PCR instrument. When the target of interest is present, the probe oligonucleotide base pairs with the amplified target. While bound, it is digested by the 5' to 3' exonuclease activity of the Taq polymerase thereby physically separating the fluorophore from the quencher and liberating signal for detection by the real time PCR instrument.

Current methods that use a unique probe for each target of interest limit the number of targets that can be measured in a single digital assay. There is a need for methods which allow for detection of multiple (e.g., three or more) targets in a single optical channel, thereby increasing the number of targets that can be measured in a single digital PCR reaction. Disclosed herein are methods and compositions for detecting three or more nucleic acid targets using digital PCR in a single optical channel. 2. Whale et al (2016) BIOMOLECULAR DETECTION AND QUANTIFICATION, vol. 10, pages 15-23, reviews multiplexing with digital PCR.

### Overview

The present disclosure provides methods for detecting more than one nucleic acid target with digital PCR in a single optical channel. In some cases, the methods of the present disclosure are used for detecting two, three, four, or more nucleic acid targets with digital PCR in a single optical channel.

In some cases, detection of multiple nucleic acid targets in a single optical channel can be achieved by providing multiple nucleic acid probes, each specific for one of multiple targets, where the probes are labeled with the same color signal tag but are provided at varying concentrations, thereby identifying each target with a unique signal readout (e.g., signal intensity value). For example, in order to detect three nucleic acid targets with digital PCR in a single optical channel, a first nucleic acid probe may be provided at a concentration of X, a second nucleic acid probe may be provided at a concentration of at least about 2X, and a third nucleic acid probe may be provided at a concentration of at least about 4X, thereby generating signals in a digital PCR reaction where each signal uniquely corresponds to the presence of each target or combination thereof in a partition.

In one example, a sample may be suspected of comprising one or more of three nucleic targets, corresponding with influenza A virus (InfA), influenza B virus (InfB), and respiratory syncytial virus B (RsvB). Unique nucleic acid primers and probes complementary to each of the three targets are provided for amplification and detection of the targets in a digital PCR reaction. Each primer set is provided at about the same concentration. Each nucleic acid probe comprises a signal tag (e.g., fluorophore) with about equal emission wavelengths, but is added to the sample at distinct concentrations to enable unique identification of each target. A probe complementary to InfA is added at a lowest concentration (e.g., X), a probe complementary to InfB is added at a moderate concentration (e.g., at least 2X), and a probe complementary to RsvB is added at a highest concentration (e.g., at least 4X). Standard digital PCR techniques are used to separate, amplify, and detect the targets, with the assumption that only one oligonucleotide molecule per target is present per droplet. The use of distinct concentrations for the oligonucleotide probes allows for the presence or absence of each target to be detected based on a given signal intensity. In this case, a signal at the lowest intensity indicates the presence of InfA, a signal of a moderate intensity indicates the presence of InfB, and a signal of the greatest intensity indicates the presence of RsvB. By this method, all three targets can be identified in a single reaction using only a single color channel. Further, through use of precise concentrations of each probe, signals corresponding with any combination of presence or absence of the three targets can be discerned. For example, where a signal intensity of 50× indicates the presence or InfA, a signal intensity of 100× indicates the presence of InfB, and a signal intensity of 200x indicates the presence of RsvB, any combination of one, two, or three targets will result in a unique signal intensity (0× = no target present, 50x = InfA only, 100× = InfB only,150× = InfA + InfB,200× = RsvB only, 250× = InfA + RsvB,300× = InfB + RsvB, 350× InfA + InfB + RsvB). In this way, it is possible to unambiguously resolve the presence or absence of the three targets, in any combination of presence or absence.

In some cases, detection of more than one nucleic acid target using a single optical channel can be achieved by providing oligonucleotide probes, each specific for one of multiple targets, where each probe is labeled with one or more copies of a luminescent signal tag, such that the probes have varying intensity levels, thereby identifying each target with a unique signal readout (e.g., signal intensity value). For example, in order to detect three nucleic acid targets with digital PCR in a single optical channel, a first nucleic acid probe having an intensity level of X may be provided, a second nucleic acid probe having an intensity level of 2X may be provided, and a third nucleic acid probe having an intensity level of 4X may be provided, thereby generating signals in a digital PCR reaction where each signal uniquely corresponds to the presence of each target and combination thereof in a partition.

In one example, a sample may be suspected of comprising three targets, InfA, InfB, and RsvB. Unique nucleic acid primers and probes complementary to each of the three targets are provided for amplification and detection of the targets in a digital PCR reaction. Each oligonucleotide probe comprises a signal tag (e.g., fluorophore) with a different intensity level to uniquely identify each target. A probe complementary to InfA comprises a luminescent signal tag with an intensity level of X, a probe complementary to InfB comprises a luminescent signal tag with an intensity level of at least about 2X, and a probe complementary to RsvB comprises a luminescent signal tag with an intensity level of at least about 4X. The use of signal tags with varying intensity levels allows for the presence or absence of each target to be detected based on a given intensity. In this case, a signal at 1× intensity indicates the presence of InfA, a signal at 2× intensity indicates the presence of InfB, and a signal at 4× intensity indicates the presence of RsvB. Similarly, a signal intensity of 3× indicates the presence of InfA + InfB, an intensity of5× indicates the presence of InfA + RsvB, an intensity of 6× indicates the presence of InfB + RsvB, and an intensity of 7× indicates the presence of InfA, InfB, and RsvB. By this method, the presence or absence of all three targets, in any combination, can be identified in a single reaction using only a single optical channel.

In addition to the use of varying intensities within a single color channel to identify multiple targets, multiple color channels can be combined to further increase the number of detectable targets in a single reaction. This can be achieved by providing probes specific to a given target which comprise different signal tags at varying concentrations and/or with varying intensity levels.

By combining the detection of more than one target within a single color channel with the use of multiple color channels, the amount of targets that can be detected in a single reaction can be increased significantly. In general, the number of targets that can be detected in a single digital PCR reaction can be equal to m^{∗}n, where m is the number of distinct intensity levels within a color channel and n is the number of color channels available. For example, where three targets can be detected within a given color channel, and where two color channels are used, nine unique targets can be identified in a single reaction. The ability to measure many targets using only a small number of color channels is a substantial improvement over existing multiplex ddPCR technologies.

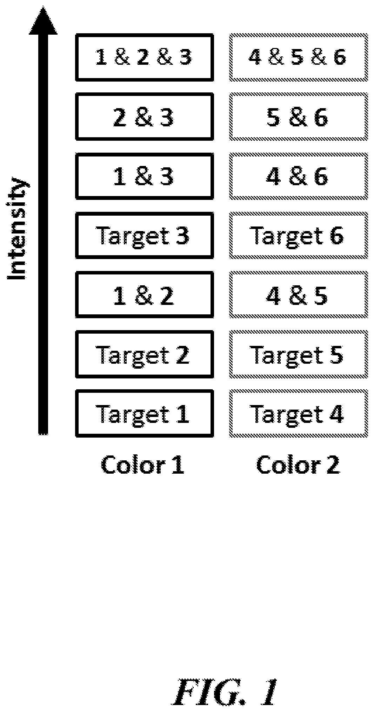

**FIG.** 1 shows a schematic representation of the signal readouts which can be used with the methods of the present disclosure to detect large numbers of nucleic acid targets in a single digital PCR reaction, where each partition contains a single target or combination of targets. Each intensity level within each color may represent a unique target or target combination, as indicated.

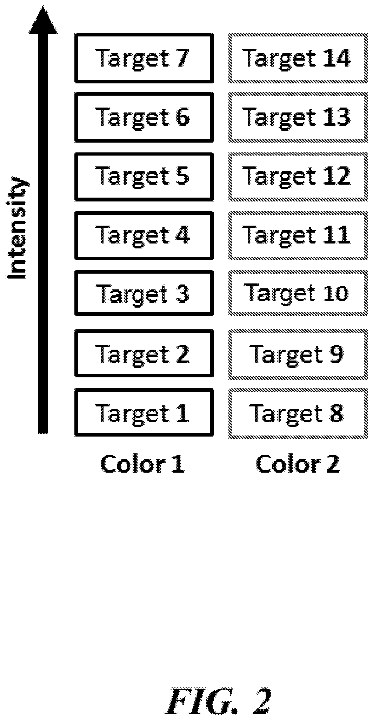

FIG. 2 shows a schematic representation of the signal readouts which can be used with the methods of the present disclosure to detect large numbers of nucleic acid targets in a single digital PCR reaction, where each partition comprises at most one nucleic acid target. Each intensity level within each color may represent a unique target, as indicated.

In some cases, each target or combination thereof is assigned a specific fluorescence signature (color combination and color intensity) by titrating the fluorogenic probes in specific ratios associated with that target. The probe and primer concentrations are chosen to be significantly higher than the target concentration, to mitigate for statistical sample partitioning variation (e.g. Poisson distribution of reagents amongst partitions).

In cases where each partition comprises at most one nucleic acid target, each partition may be measured individually, and its fluorescence signature used to identify the target present in that partition. Table 1 shows reagent concentrations for an example digital assay which can be used to accomplish the following signal output for multiple targets:
• 1× signal level in channel 1 and a 0× signal level in channel 2 identifies Target A
• 2× signal level in channel 1 and a 0× signal level in channel 2 identifies Target B
• 0× signal level in channel 1 and a 1× signal level in channel 2 identifies Target C
• 1× signal level in channel 1 and a 1× signal level in channel 2 identifies Target D

**Table 1**

| | Probe Color 1 | Probe Color 2 | Forward Primer | Reverse Primer |
|---|---|---|---|---|
| Target A | 300nM | 0nM | 1200nM | 1200nM |
| Target B | 600nM | 0nM | 1200nM | 1200nM |
| Target C | 0nM | 300nM | 1200nM | 1200nM |
| Target D | 300nM | 300nM | 1200nM | 1200nM |

### Digital Assays

In some aspects, the present disclosure provides methods for performing a digital assay. A method for performing a digital assay may comprise partitioning a plurality of nucleic acid targets and a plurality of nucleic acid probes into a plurality of partitions. In some cases, two, three, four, five, or more nucleic acid targets may be partitioned into a plurality of partitions together with two, three, four, five, six, seven, eight, nine, ten, 11, 12, 13, 14, 15, 16, 17, 18, 19, 20 or more nucleic acid probes. Following partitioning, the nucleic acid targets may be amplified in the partitions, for example, by polymerase chain reaction (PCR). Next, N signals may be generated from the nucleic acid probes. Each signal of the N signals may correspond to the presence of a unique combination of nucleic acid targets in a partition. Following signal generation, the N signals may be detected in a single optical channel. The signals may be detected using, for example, fluorescence detection in a single color channel.

A method for performing a digital assay may comprise amplifying nucleic acid targets from a sample in a plurality of partitions comprising nucleic acid probes complementary to the nucleic acid targets. Each nucleic acid probe may be labeled with a fluorophore. The fluorophores may be capable of being detected in a single optical channel. For example, the fluorophores may each comprise similar emission wavelength spectra, such that they can be detected in a single optical channel. Following partitioning, N signals may be detected from the plurality of partitions if one or more of the nucleic acid targets is present. Each of the N signals may correspond to a unique combination of one or more of the nucleic acid targets present in a partition. From the N signals, the presence or absence of each of the nucleic acid targets in the sample may be determined.

In some cases, the concentrations of each of the nucleic acid probes provided in the disclosed methods are such that each signal generated from the nucleic acid probes uniquely identifies a specific combination of nucleic acid targets in a partition. For example, where three nucleic acid probes are provided, a first nucleic acid probe may be provided at a concentration of about X, a second nucleic acid probe may be provided at a concentration of at least about 2X, and a third nucleic acid probe may be provided at a concentration of at least about 4X, such that a signal generated in a partition corresponds to a unique combination of nucleic acid targets in the partition. Where four nucleic acid probes are provided, a first nucleic acid probe may be provided at a concentration of about X, a second nucleic acid probe may be provided at a concentration of at least about 2X, a third nucleic acid probe may be provided at a concentration of at least about 4X, and a fourth nucleic acid probe may be provided at a concentration of at least about 8X, such that a signal generated in a partition corresponds to a unique combination of nucleic acid targets in the partition. Various combinations of nucleic acid probe concentrations may be used in the disclosed methods, as described elsewhere herein.

In some cases, the intensity levels of each of the nucleic acid probes provided in the disclosed methods are such that the signals generated from the nucleic acid probes uniquely identify a specific combination of nucleic acid targets in a partition. For example, where three nucleic acid probes are provided, a first nucleic acid probe may have an intensity level of about Y, a second nucleic acid probe may have an intensity level of at least about 2Y, and a third nucleic acid probe may have an intensity level of at least about 4Y. Where four nucleic acid probes are provided, a first nucleic acid probe may have an intensity level of about Y, a second nucleic acid probe may have an intensity level of at least about 2Y, and a third nucleic acid probe may have an intensity level of at least about 4Y, and a fourth nucleic acid probe may have an intensity level of at least about 8Y. Each nucleic acid probe may comprise one fluorophore having various intensity levels, such that each nucleic acid probe has a different intensity level. Alternatively or in addition, each nucleic acid probe may comprise multiple fluorophores, each having about the same intensity level, such that each nucleic acid probe has a different intensity level.

### Amplification

Amplification of nucleic acid targets may be performed using a suitable amplification method. Amplification may comprise linear amplification. Amplification may comprise polymerase chain reaction. Amplification may comprise a nucleic acid extension reaction. Amplification may be performed before or after partitioning. In some cases, amplification of nucleic acid targets is performed in a plurality of partitions. For example, nucleic acid targets may be partitioned into a plurality of droplets, and amplification performed within each droplet, thereby generating a signal if at least one nucleic acid target is present in a droplet.

Amplification conditions may be modified such that the signals generated in a digital assay uniquely identify each combination of targets. Amplification conditions may comprise thermal cycling conditions, including temperature and length in time of each thermal cycle. The use of particular amplification conditions may serve to modify the signal intensity of each signal, thereby enabling each signal to correspond to a unique combination of nucleic acid targets in a partition.

### Signals

A signal may be a fluorescent signal. A signal may correspond to a fluorescence intensity level. Each signal measured in the methods of the present disclosure may have a distinct fluorescence intensity value, thereby corresponding to the presence of a unique combination of nucleic acid targets in a partition. A signal may be generated by one or more nucleic acid probes. The number of signals generated in a digital assay may correspond to the number of nucleic acid probes that are partitioned, the number of nucleic acid targets that are partitioned, and, in some cases, the partitioning conditions. For example, where three nucleic acid targets and three nucleic acid probes are partitioned such that each partition may comprise one, two, or all three nucleic acid targets, seven signals may be generated, where each signal corresponds to the presence of a unique combination of the three nucleic acid targets in the partition. N may be a number of signals detected in a single optical channel in a digital assay of the present disclosure. N may be at least 2, 3, 4, 5, 6, 7, 8, 9, 10, 11, 12, 13, 14, 15, 20, 25, 30, 40, 50 or more. N may be at most 50, 40, 30, 24, 20, 15, 14, 13, 12, 11, 10, 9, 8, 7, 6, 5, 4, 3, or 2. N may be 2, 3, 4, 5, 6, 7, 8, 9, 10, 11, 12, 13, 14, 15, 20, 25, 30, 40, or 50. As will be recognized and is described elsewhere herein, sets of signals may be generated in multiple different optical channels, where each set of signals is detected in a single optical channel, thereby significantly increasing the number of nucleic acid targets that can be measured in a single digital PCR reaction. In some cases, two sets of signals are detected in a single reaction. Each set of signals detected in a reaction may comprise the same number of signals, or different numbers of signals.

### Partitioning

Methods of the present disclosure may comprise partitioning nucleic acid targets, nucleic acid probes, and, in some cases, additional reagents into a plurality of partitions. A partition may be a droplet. A partition may be an emulsion. A partition may be a well. A partition may be a microwell. Partitioning may be performed using a microfluidic device. In some cases, partitioning is performed using a droplet generator. Partitioning may comprise dividing a mixture (e.g., a mixture comprising nucleic acid targets, and nucleic acid probes) into water-in-oil droplets. A droplet may comprise one or more nucleic acid targets. A droplet may comprise a single nucleic acid target. A droplet may comprise two or more nucleic acid targets. A droplet may comprise no nucleic acid targets.

### Nucleic acid probes

Any number of different nucleic acid probes may be partitioned together with nucleic acid targets in the disclosed methods. In some cases, 3, 4, 5, 6, 7, 8, 9, 10, 11, 12, 13, 14, 15, 20, 30, 40, 50, or more different nucleic acid probes are partitioned into a plurality of partitions. Each probe may be specific for (e.g., capable of binding to) a given nucleic acid target in a sample. For example, three nucleic acid probes may be provided comprising a first nucleic acid probe, a second nucleic acid probe, and a third nucleic acid probe, where the first nucleic acid probe is specific for a first nucleic acid target, the second nucleic acid probe is specific for a second nucleic acid target, and a third nucleic acid probe is specific for a third nucleic acid target. Each nucleic acid probe may comprise a signal tag with about equal emission wavelengths. In some cases, each nucleic acid probe comprises an identical fluorophore. In some cases, each nucleic acid probe comprises a different fluorophore, where each fluorophore is capable of being detected in a single optical channel.

Each nucleic acid probe of a plurality of nucleic acid probes may be provided at a specific concentration, such that each signal generated from the nucleic acid probes in a partition corresponds to a unique combination of target nucleic acids. Where each signal of N signals corresponds to a unique combination of target nucleic acids, the signals may be described as "non-degenerate". In one aspect, a first nucleic acid probe is provided at a concentration of about X. Where a first nucleic acid probe is provided at a concentration of about X, additional nucleic acid probes may be provided at a specific concentration greater than X, thereby enabling the generation of non-degenerate signals.

In some cases, a second nucleic acid probe is provided at a concentration of at least about 2X, about 3X, about 4X, about 5X, about 6X, about 7X, about 8X, or more. In some cases, a second nucleic acid probe is provided at a concentration of at most about 8X, about 7X, about 6X, about 5X, about 4X, about 3X, or about 2X. In some cases, a second nucleic acid probe is provided at a concentration of about 2X, about 3X, about 4X, about 5X, about 6X, about 7X, or about 8X.

In some cases, a third nucleic acid probe is provided at a concentration of at least about 4X, about 5X, about 6X, about 7X, about 8X, about 9X, about 10X, about 11X, about 12X or more. In some cases, a third nucleic acid probe is provided at a concentration of at most about 12X, about 11X, about 10X, about 9X, about 8X, about 7X, about 6X, about 5X, or about 4X. In some cases, a third nucleic acid probe is provided at a concentration of about 4X, about 5X, about 6X, about 7X, about 8X, about 9X, about 10X, about 11X, or about 12X.

In some cases, a fourth nucleic acid probe is provided at a concentration of at least about 8X, about 9X, about 10X, about 11X, about 12X, about 13X, about 14X, about 15X, about 20X, or more. In some cases, a fourth nucleic acid probe is provided at a concentration of at most about 20X, about 15X, about 14X, about 13X, about 12X, about 11X, about 10X, about 9X, or about 8X. In some cases, a fourth nucleic acid probe is provided at a concentration of about 8X, about 9X, about 10X, about 11X, about 12X, about 13X, about 14X, about 15X, or about 20X.

Each nucleic acid probe may be provided at a concentration that is distinct from all other nucleic acid probes. Any combination of nucleic acid probe concentrations which is capable of producing non-degenerate signals (i.e., signals which each uniquely identify a specific combination of nucleic acid targets in a partition) may be used. In one example, a first nucleic acid probe may be provided at a concentration of about X, a second nucleic acid probe may be provided at a concentration of about 3X, and a third nucleic acid probe may be provided at a concentration of about 8X, thereby generating non-degenerate signals in a plurality of partitions corresponding to the presence of a unique combination of nucleic acid targets.

X may be a concentration of a nucleic acid probe provided in the disclosed methods. In some cases, X is at least 50 nM, 100 nM, 150 nM, 200 nM, 250 nM, 300 nM, 350 nM, 400 nM, 450 nM, 500 nM, or greater. In some cases, X is at most 500 nM, 450 nM, 400 nM, 350 nM, 300 nM, 250 nM, 200 nM, 150 nM, 100 nM, or 50 nM. X may be any concentration of a nucleic acid probe which is capable of being partitioned.

Each nucleic acid probe of a plurality of nucleic acid probes may be labeled with one or more copies of a luminescent signal tag, such that the probes have varying intensity levels, thereby enabling the generation of signals which uniquely identify each combination of nucleic acid targets in a partition. In one aspect, a first nucleic acid probe has an intensity level of about Y. Where a first nucleic acid probe has an intensity level of about Y, additional nucleic acid probes may have an intensity level greater than Y, thereby enabling the generation of non-degenerate signals. A first nucleic acid may comprise a fluorophore with an intensity level of about Y.

In some cases, a second nucleic acid probe has an intensity level of at least about 2Y, about 3Y, about 4Y, about 5Y, about 6Y, about 7Y, about 8Y, or more. In some cases, a second nucleic acid probe has an intensity level of at most about 8Y, about 7Y, about 6Y, about 5Y, about 4Y, about 3Y, or about 2Y. In some cases, a second nucleic acid probe has an intensity level of about 2Y, about 3Y, about 4Y, about 5Y, about 6Y, about 7Y, or about 8Y. A second nucleic acid may comprise a single luminescent signal tag (e.g., fluorophore) with an intensity level of at least about 2Y. Alternatively or in addition, a second nucleic acid may comprise two or more fluorophores each with an intensity level of about Y.

In some cases, a third nucleic acid probe has an intensity level of at least about 4Y, about 5Y, about 6Y, about 7Y, about 8Y, about 9Y, about 10Y, about 11Y, about 12Y or more. In some cases, a third nucleic acid probe has an intensity level of at most about 12Y, about 11Y, about 10Y, about 9Y, about 8Y, about 7Y, about 6Y, about 5Y, or about 4Y. In some cases, a third nucleic acid probe has an intensity level of about 4Y, about 5Y, about 6Y, about 7Y, about 8Y, about 9Y, about 10Y, about 11Y, or about 12Y. A third nucleic acid may comprise a single luminescent signal tag (e.g., fluorophore) with an intensity level of at least about 4Y. Alternatively or in addition, a third nucleic acid may comprise four or more fluorophores each with an intensity level of about Y.

In some cases, a fourth nucleic acid probe has an intensity level of at least about 8Y, about 9Y, about 10Y, about 11Y, about 12Y, about 13Y, about 14Y, about 15Y, about 20Y, or more. In some cases, a fourth nucleic acid probe has an intensity level of at most about 20Y, about 15Y, about 14Y, about 13Y, about 12Y, about 11Y, about 10Y, about 9Y, or about 8Y. In some cases, a fourth nucleic acid probe has an intensity level of about 8Y, about 9Y, about 10Y, about 11Y, about 12Y, about 13Y, about 14Y, about 15Y, or about 20Y. A fourth nucleic acid may comprise a single luminescent signal tag (e.g., fluorophore) with brightness of at least about 8Y. Alternatively or in addition, a fourth nucleic acid may comprise eight or more fluorophores each with an intensity level of about Y.

Y may be a signal intensity level produced by a nucleic acid probe comprising one or more signal tags (e.g., fluorophores). Y may be any signal intensity capable of being detected in an optical channel.

A nucleic acid probe may be a nucleic acid complementary to a region of a given nucleic acid target. A nucleic acid probe may comprise a signal tag. A signal tag may comprise a means of generating a signal such as, for example, a fluorophore. The fluorophore may be, for example, FAM, TET, HEX, JOE, Cy3, or Cy5. A nucleic acid probe may further comprise one or more quenchers. A quencher may inhibit signal generation from a fluorophore. A quencher may be, for example, TAMRA, BHQ-1, BHQ-2, or Dabcy.

### Samples and targets

A nucleic acid target of the present disclosure may be derived from any source including, for example, viruses, bacterial cells, and eukaryotic cells. A nucleic acid target may be derived from one or more cells. A cell may be a tumor cell. A cell may be a cell suspected of comprising a viral pathogen. In some cases, a nucleic acid target is derived from a cell-free sample (e.g., serum, plasma). A nucleic acid target may be cell-free nucleic acid. Cell-free nucleic acid may be, for example, cell-free tumor DNA, cell-free fetal DNA, cell-free RNA, etc. A nucleic acid target may comprise deoxyribonucleic acid (DNA). DNA may be any kind of DNA, including genomic DNA. A nucleic acid target may be viral DNA. A nucleic acid target may comprise ribonucleic acid (RNA). RNA may be any kind of RNA, including messenger RNA, transfer RNA, ribosomal RNA, and microRNA. RNA may be viral RNA. A nucleic acid target may comprise a gene whose detection may be useful in diagnosing one or more diseases. A gene may be a viral gene or bacterial gene whose detection may be useful in identifying the presence or absence of a pathogen in a subject. In some cases, the methods of the present disclosure are useful in detecting the presence or absence or one or more infectious agents (e.g., viruses) in a subject. In some cases, the methods of the present disclosure are useful in detecting the relative amount of a fetal nucleic acid in a cell-free nucleic acid sample from a subject, thereby diagnosing the fetus for one or more genetic abnormalities. In some cases, the methods of the present disclosure are useful in detecting the presence or absence of tumor DNA in a cell-free nucleic acid sample from a subject, thereby diagnosing the subject for cancer.

### EXAMPLES

### Example 1

A mixture of primers, nucleic acid probes, and polymerase was added to a sample containing synthetic DNA targets for Pan InfA, InfB, and RsvB at equal concentrations of 10,000 copies per reaction. The concentration of each primer, nucleic acid probe, and polymerase is shown in Table 2.

**Table 2**

| **Reagent** | **Concentration** |
|---|---|
| Pan InfA forward primer | 3.0 µM |
| Pan InfA reverse primer | 3.0 µM |
| Pan InfA probe | 200 nM |
| InfB forward primer | 3.0 µM |
| InfB reverse primer | 3.0 µM |
| InfB probe | 400 nM |
| RsvB forward primer | 3.0 µM |
| RsvB reverse primer | 3.0 µM |
| RsvB probe | 1.6 µM |
| Bio-Rad Digital PCR polymerase enzyme | 2 units |

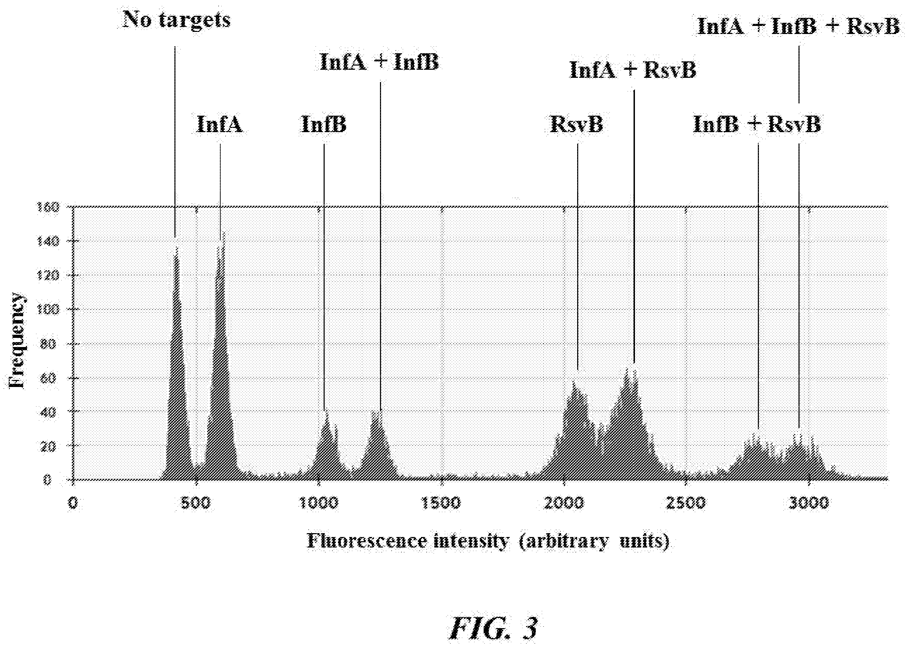

24 µL of this total reaction volume was dispersed into 80 µL of digital droplet PCR oil, generating a population of approximately 15,000 droplets. These droplets were thermally cycled using a Bio-Rad C1000 thermal cycler with the following PCR protocol: 95°C hotstart for 10min; 35 cycles at 95°C denature for 30 seconds and 57°C annealing/extension for 120 seconds. Following PCR, the droplets were then measured using the Bio-Rad QX200 droplet reader. The results of this experiment are shown in **FIG. 3**. The presence of all three targets in the ddPCR reaction mixture generated eight distinct population clusters of droplets, ranging from clusters with no targets (intensity peak of 350 AFU; "No Targets"), to clusters will all targets (intensity peak of 2800 AFU; "InfA + InfB + RsvB") as shown in **FIG. 3**. These data illustrate the ability of the disclosed methods to resolve multiple targets in a single-color channel of a digital PCR instrument.

### Example 2

A probe dilution series was performed using primers, probes, and synthetic templates for a single human rhinovirus (HRV) target. The concentrations of the reactants (primers, probes, and templates) for each experiment are listed in Table 3. Seven experiments were performed (a-g), as marked, each with a different concentration of HRV probe.

**Table 3**

| **Experiment** | **HRV Forward Primer** | **HRV Reverse Primer** | **HRV Probe** | **HRV Target** |
|---|---|---|---|---|
| a | 10.0 µM | 10.0 µM | 0 nM | 50,000 copies/rxn |
| b | 10.0 µM | 10.0 µM | 100 nM | 50,000 copies/rxn |
| c | 10.0 µM | 10.0 µM | 200 nM | 50,000 copies/rxn |
| d | 10.0 µM | 10.0 µM | 500 nM | 50,000 copies/rxn |
| e | 10.0 µM | 10.0 µM | 1.0 µM | 50,000 copies/rxn |
| f | 10.0 µM | 10.0 µM | 2.0 µM | 50,000 copies/rxn |
| g | 10.0 µM | 10.0 µM | 5.0 µM | 50,000 copies/rxn |

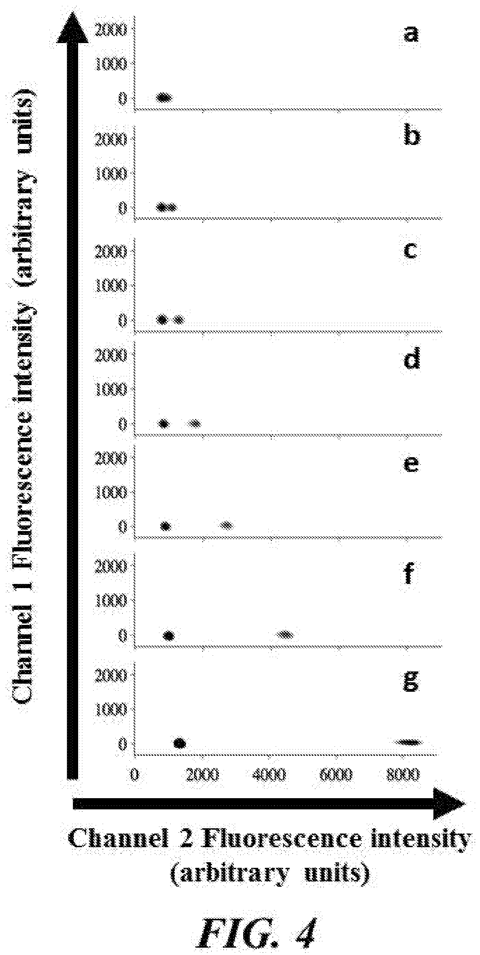

These reactants were added to a ddPCR polymerase enzyme mixture. 21 µL of this total reaction volume was dispersed into 80 µL of digital droplet PCR oil, generating a population of approximately 1,000 droplets. These droplets were thermally cycled using a Bio-Rad C1000 thermal cycler with the following PCR protocol: 95°C hotstart for 10min; 35 cycles at 95°C denature for 30 seconds and 57°C annealing/extension for 120 seconds. Following PCR, the droplets were then measured using the Bio-Rad QX200 droplet reader. The results of these experiments are shown in **FIG. 4**. Each reaction generated two population clusters of droplets: one at low intensity for droplets containing no amplified targets, and one at a higher intensity containing droplets with amplified target, as shown in **FIG. 4**. In experiment a, the population of empty and target-occupied droplets could not be distinguished, as no reporting probes were added, serving as a negative control. For all other experiments, distinct populations were observed for empty and target-occupied droplets. The signal intensity of the target-occupies droplets increased with increasing HRV probe concentration. These data highlight the large optical dynamic range of the disclosed multiplexing ddPCR methods.

### Example 3

Targets for RsvA, RsvB, and HRV were labeled with TaqMan probes fluorescing in the FAM channel, while targets for the Pan InfA markers as well as the H1 and H3 serotypes were labeled with probes fluorescing in the VIC channel. The primer and probe mixture was designed to generate signatures specific to the presences or absence of each target. For each experiment, 8µL of the primer mixture was added to 12µL of a Bio-Rad Digital PCR polymerase enzyme master mix and 6µL of sample volume, for a total reaction volume of 24µL. The number of copies used in each reaction is shown in Table 4.

**Table 4**

| **Experiment** | **RsvA** | **HRV** | **RsvB** | **Pan InfA** | **InfA H1** | **InfA H3** |
|---|---|---|---|---|---|---|
| a | 10,000 copies/rxn | - | - | - | - | - |
| b | - | - | - | 10,000 copies/rxn | - | 10,000 copies/rxn |
| c | - | - | 10,000 copies/rxn | 10,000 copies/rxn | 10,000 copies/rxn | 10,000 copies/rxn |
| d | 10,000 copies/rxn | 10,000 copies/rxn | 10,000 copies/rxn | 10,000 copies/rxn | - | - |
| e | 10,000 copies/rxn | 10,000 copies/rxn | 10,000 copies/rxn | 10,000 copies/rxn | 10,000 copies/rxn | 10,000 copies/rxn |

This volume was dispersed in 80µL of droplet reader oil and thermally cycled under the following conditions: 95°C hotstart for 10min; 35 cycles at 95°C denature for 30 seconds and 57°C annealing/extension for 120 seconds. The droplets were then read using Bio-Rad QX200 droplet reader.

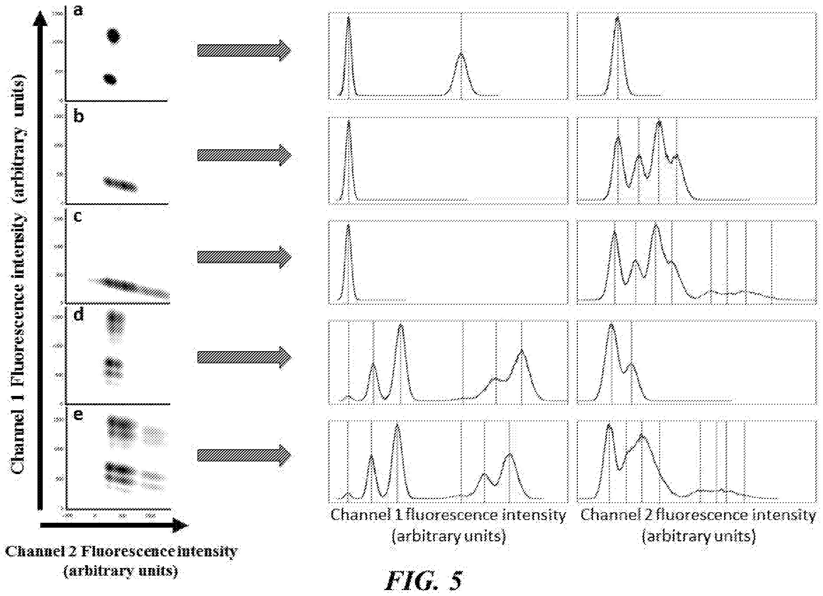

The results of these experiments are shown in **FIG. 5**. Two-dimensional signal readouts are shown on the left for each experiment (a-e, as marked), with corresponding one-dimensional signal readouts for each optical channel on the right. Each signal identifying a unique combination of nucleic acid targets in the reaction is labeled with a vertical line. These data demonstrate the detection of six targets across two color channels in a single ddPCR reaction.

The term "about" when referring to a measurable value such as an amount, a temporal duration, and the like, is meant to encompass variations of ±20% or in some instances ±10%, or in some instances ±5%, or in some instances ±1%, or in some instances ±0.1 % from the specified value, as such variations are appropriate to perform the disclosed methods. Further, "about" can mean plus or minus less than 1 or 1, 2, 3, 4, 5, 6, 7, 8, 9, 10, 11, 12, 13, 14, 15, 16, 17, 18, 19, 20, 25, 30, or greater than 30 percent, depending upon the situation and known or knowable by one skilled in the art. About also includes the exact amount. Hence "about 200 nM" means "about 200 nM" and also "200 nM".

## Claims

1. A method for performing a digital assay, comprising:
(a) partitioning into a plurality of partitions (i) three or more nucleic acid targets and (ii) a set of nucleic acid probes comprising a first nucleic acid probe, a second nucleic acid probe, and a third nucleic acid probe;
(b) amplifying the three or more nucleic acid targets in the partitions;
(c) generating N signals from the set of nucleic acid probes, each of which N signals corresponds to the presence of a unique combination of nucleic acid targets of the three or more nucleic acid targets in a partition of the plurality of partitions; and
(d) detecting the N signals in a single optical channel; wherein, in (a), the first nucleic acid probe, second nucleic acid probe and third nucleic acid probe are configured such that (i) the concentration of the first nucleic acid probe is about X, the concentration of the second nucleic acid probe is at least about 2X, and the concentration of the third nucleic acid probe is at least about 4X, or (ii) the first nucleic acid probe has an intensity level of about Y, the second nucleic acid probe has an intensity level of at least about 2Y and the third nucleic acid probe has an intensity level of at least about 4Y.

2. The method of claim 1, wherein N is 3 or more.

3. The method of claim 1 or claim 2, wherein N is 4 or more.

4. The method of any of claims 1 to 3, wherein the three or more nucleic acid targets are four or more nucleic acid targets.

5. The method of any preceding claim, wherein the set of nucleic acid probes further comprises a fourth nucleic acid probe.

6. The method of claim 5, wherein, in (a), the concentration of the first nucleic acid probe is about X, the concentration of the second nucleic acid probe is at least about 2X, the concentration of the third nucleic acid probe is at least about 4X and the concentration of the fourth nucleic acid probe is at least about 8X.

7. The method of any preceding claim, wherein each of the N signals corresponds to a fluorescence intensity level.

8. The method of any preceding claim, wherein each of the set of nucleic acid probes comprises an identical fluorophore.

9. The method of any of claims 1 to 7, wherein each of the set of nucleic acid probes comprises a different fluorophore.

10. The method of any preceding claim, wherein the amplifying comprises linear amplification.

11. The method of claim 1, wherein the first nucleic acid probe has an intensity level of about Y, the second nucleic acid probe has an intensity level of at least about 2Y, and the third nucleic acid probe has an intensity level of at least about 4Y; and wherein the first nucleic acid probe comprises one fluorophore with an intensity level of about Y, the second nucleic acid probe comprises at least two fluorophores each with an intensity level of about Y, and the third nucleic acid probe comprises at least four fluorophores each with an intensity level of about Y.

12. The method of claim 1, wherein the first nucleic acid probe has an intensity level of about Y, the second nucleic acid probe has an intensity level of at least about 2Y, and the third nucleic acid probe has an intensity level of at least about 4Y; and wherein the first nucleic acid probe comprises a fluorophore with an intensity level of about Y, the second nucleic acid probe comprises a fluorophore with an intensity level of at least about 2Y, and the third nucleic acid probe comprises a fluorophore with an intensity level of at least about 4Y.

13. The method of claim 5, wherein the first nucleic acid probe has an intensity level of about Y, the second nucleic acid probe has an intensity level of at least about 2Y, and the third nucleic acid probe has an intensity level of at least about 4Y; and the fourth nucleic acid probe has an intensity level of at least about 8Y.

14. The method of claim 13, wherein the fourth nucleic acid probe comprises a fluorophore with an intensity level of at least about 8Y.

15. The method of any preceding claim, wherein the plurality of partitions is a plurality of droplets.

## Patentansprüche

1. Verfahren zur Durchführung eines digitalen Assay, wobei das Verfahren folgendes umfasst:
(a) Partitionieren in eine Mehrzahl von Partitionen von (i) drei oder mehr Nukleinsäurezielen und (ii) einer Menge von Nukleinsäuresonden, die eine erste Nukleinsäuresonde, eine zweite Nukleinsäuresonde und eine dritte Nukleinsäuresonde umfasst;
(b) Amplifizieren der drei oder mehr Nukleinsäureziele in den Partitionen;
(c) Erzeugen von N Signalen aus der Menge von Nukleinsäuresonden, wobei jedes der N Signale der Präsenz einer einzigartigen Kombination von Nukleinsäurezielen der drei oder mehr Nukleinsäureziele in einer Partition der Mehrzahl von Partitionen entspricht; und
(d) Nachweisen der N Signale in einem einzigen optischen Kanal;
wobei die erste Nukleinsäuresonde, die zweite Nukleinsäuresonde und die dritte Nukleinsäuresonde in (a) so gestaltet sind, dass (i) die Konzentration der ersten Nukleinsäuresonde etwa X beträgt, die Konzentration der zweiten Nukleinsäuresonde mindestens etwa 2X beträgt und die Konzentration der dritten Nukleinsäuresonde mindestens etwa 4X beträgt, oder dass (ii) die erste Nukleinsäuresonde einen Intensitätswert von etwa Y aufweist, die zweite Nukleinsäuresonde einen Intensitätswert von mindestens etwa 2Y aufweist und die dritte Nukleinsäuresonde einen Intensitätswert von mindestens etwa 4Y aufweist.

2. Verfahren nach Anspruch 1, wobei N 3 oder mehr beträgt.

3. Verfahren nach Anspruch 1 oder Anspruch 2, wobei N 4 oder mehr beträgt.

4. Verfahren nach einem der Ansprüche 1 bis 3, wobei die drei oder mehr Nukleinsäureziele vier oder mehr Nukleinsäureziele sind.

5. Verfahren nach einem der vorstehenden Ansprüche, wobei die Menge von Nukleinsäuresonden ferner eine vierte Nukleinsäuresonde umfasst.

6. Verfahren nach Anspruch 5, wobei in (a) die Konzentration der ersten Nukleinsäuresonde etwa X beträgt, die Konzentration der zweiten Nukleinsäuresonde mindestens etwa 2X beträgt, die Konzentration der dritten Nukleinsäuresonde mindestens etwa 4X beträgt und die Konzentration der vierten Nukleinsäuresonde mindestens etwa 8X beträgt.

7. Verfahren nach einem der vorstehenden Ansprüche, wobei jedes der N Signale einem Fluoreszenzintensitätswert entspricht.

8. Verfahren nach einem der vorstehenden Ansprüche, wobei jede Nukleinsäuresonde der Menge von Nukleinsäuresonden ein identisches Fluorophor umfasst.

9. Verfahren nach einem der Ansprüche 1 bis 7, wobei jede Nukleinsäuresonde der Menge von Nukleinsäuresonden ein anderes Fluorophor umfasst.

10. Verfahren nach einem der vorstehenden Ansprüche, wobei das Amplifizieren eine lineare Amplifikation umfasst.

11. Verfahren nach Anspruch 1, wobei die erste Nukleinsäuresonde einen Intensitätswert von etwa Y aufweist, die zweite Nukleinsäuresonde einen Intensitätswert von mindestens etwa 2Y aufweist und die dritte Nukleinsäuresonde einen Intensitätswert von mindestens etwa 4Y aufweist; und wobei die erste Nukleinsäuresonde ein Fluorophor mit einem Intensitätswert von etwa Y aufweist, die zweite Nukleinsäuresonde mindestens zwei Fluorophore mit einem Intensitätswert von jeweils etwa Y aufweist und die dritte Nukleinsäuresonde mindestens vier Fluorophore mit einem Intensitätswert von jeweils etwa Y aufweist.

12. Verfahren nach Anspruch 1, wobei die erste Nukleinsäuresonde einen Intensitätswert von etwa Y aufweist, die zweite Nukleinsäuresonde einen Intensitätswert von mindestens etwa 2Y aufweist und die dritte Nukleinsäuresonde einen Intensitätswert von mindestens etwa 4Y aufweist; und wobei die erste Nukleinsäuresonde ein Fluorophor mit einem Intensitätswert von etwa Y aufweist, die zweite Nukleinsäuresonde ein Fluorophor mit einem Intensitätswert von mindestens etwa 2Y aufweist und die dritte Nukleinsäuresonde ein Fluorophor mit einem Intensitätswert von mindestens etwa 4Y aufweist.

13. Verfahren nach Anspruch 5, wobei die erste Nukleinsäuresonde einen Intensitätswert von etwa Y aufweist, die zweite Nukleinsäuresonde einen Intensitätswert von mindestens etwa 2Y aufweist und die dritte Nukleinsäuresonde einen Intensitätswert von mindestens etwa 4Y aufweist; und wobei die vierte Nukleinsäuresonde einen Intensitätswert von mindestens etwa 8Y aufweist.

14. Verfahren nach Anspruch 13, wobei die vierte Nukleinsäuresonde ein Fluorophor mit einem Intensitätswert von mindestens etwa 8Y aufweist.

15. Verfahren nach einem der vorstehenden Ansprüche, wobei die Mehrzahl von Partitionen eine Mehrzahl von Tröpfchen ist.

## Revendications

1. Procédé pour effectuer un dosage numérique, comprenant les étapes consistant à :
(a) séparer en une pluralité de séparations (i) trois cibles d'acide nucléique ou plus et (ii) un ensemble de sondes nucléiques comprenant une première sonde nucléique, une deuxième sonde nucléique et une troisième sonde nucléique ;
(b) amplifier les trois cibles d'acide nucléique ou plus dans les séparations ;
(c) générer N signaux à partir de l'ensemble de sondes nucléiques, chacun de ces N signaux correspondant à la présence d'une combinaison unique de cibles d'acide nucléique parmi les trois cibles d'acide nucléique ou plus dans une séparation de la pluralité de séparations ; et
(d) détecter les N signaux dans un seul canal optique ;
dans (a), la première sonde nucléique, la deuxième sonde nucléique et la troisième sonde nucléique étant conçues de sorte que (i) la concentration de la première sonde nucléique soit d'environ X, la concentration de la deuxième sonde nucléique soit d'au moins environ 2X, et la concentration de la troisième sonde nucléique soit d'au moins environ 4X, ou (ii) la première sonde nucléique ait un niveau d'intensité d'environ Y, la deuxième sonde nucléique ait un niveau d'intensité d'au moins environ 2Y et la troisième sonde nucléique ait un niveau d'intensité d'au moins environ 4Y.

2. Procédé selon la revendication 1, N étant 3 ou plus.

3. Procédé selon la revendication 1 ou la revendication 2, N étant 4 ou plus.

4. Procédé selon l'une quelconque des revendications 1 à 3, les trois cibles d'acide nucléique ou plus étant quatre cibles d'acide nucléique ou plus.

5. Procédé selon l'une quelconque des revendications précédentes, l'ensemble de sondes nucléiques comprenant en outre une quatrième sonde nucléique.

6. Procédé selon la revendication 5, dans (a), la concentration de la première sonde nucléique étant d'environ X, la concentration de la deuxième sonde nucléique étant d'au moins environ 2X, la concentration de la troisième sonde nucléique étant d'au moins environ 4X et la concentration de la quatrième sonde nucléique étant d'au moins environ 8X.

7. Procédé selon l'une quelconque des revendications précédentes, chacun des N signaux correspondant à un niveau d'intensité de fluorescence.

8. Procédé selon l'une quelconque des revendications précédentes, chaque sonde de l'ensemble de sondes nucléiques comprenant un fluorophore identique.

9. Procédé selon l'une quelconque des revendications 1 à 7, chaque sonde de l'ensemble de sondes nucléiques comprenant un fluorophore différent.

10. Procédé selon l'une quelconque des revendications précédentes, l'amplification comprenant une amplification linéaire.

11. Procédé selon la revendication 1, la première sonde nucléique ayant un niveau d'intensité d'environ Y, la deuxième sonde nucléique ayant un niveau d'intensité d'au moins environ 2Y, et la troisième sonde nucléique ayant un niveau d'intensité d'au moins environ 4Y ; et la première sonde nucléique comprenant un fluorophore avec un niveau d'intensité d'environ Y, la deuxième sonde nucléique comprenant au moins deux fluorophores ayant chacun un niveau d'intensité d'environ Y, et la troisième sonde nucléique comprenant au moins quatre fluorophores ayant chacun un niveau d'intensité d'environ Y.

12. Procédé selon la revendication 1, la première sonde nucléique ayant un niveau d'intensité d'environ Y, la deuxième sonde nucléique ayant un niveau d'intensité d'au moins environ 2Y, et la troisième sonde nucléique ayant un niveau d'intensité d'au moins environ 4Y ; et la première sonde nucléique comprenant un fluorophore avec un niveau d'intensité d'environ Y, la deuxième sonde nucléique comprenant un fluorophore avec un niveau d'intensité d'au moins environ 2Y, et la troisième sonde nucléique comprenant un fluorophore avec un niveau d'intensité d'au moins environ 4Y.

13. Procédé selon la revendication 5, la première sonde nucléique ayant un niveau d'intensité d'environ Y, la deuxième sonde nucléique ayant un niveau d'intensité d'au moins environ 2Y, et la troisième sonde nucléique ayant un niveau d'intensité d'au moins environ 4Y ; et la quatrième sonde nucléique ayant un niveau d'intensité d'au moins environ 8Y.

14. Procédé selon la revendication 13, la quatrième sonde nucléique comprenant un fluorophore avec un niveau d'intensité d'au moins environ 8Y.

15. Procédé selon l'une quelconque des revendications précédentes, la pluralité de séparations étant une pluralité de gouttelettes.
